(19) Europäisches Patentamt
European Patent Office
Office européen des brevets

(11) Publication number: **0 393 190 A1**

(12) # EUROPEAN PATENT APPLICATION published in accordance with Art. 158(3) EPC

(21) Application number: 89901345.2

(22) Date of filing: 26.10.88

(86) International application number: PCT/SU88/00210

(87) International publication number: WO 90/04611 (03.05.90 90/10)

(51) Int. Cl.5: C08F 216/06, C08F 8/28, A61K 31/765, A61L 15/03, A61L 15/06

(43) Date of publication of application: 24.10.90 Bulletin 90/43

(84) Designated Contracting States: BE CH DE FR GB IT LI LU NL

(71) Applicant: SOROKIN, Anatoly Yakovlevich
Tikhoretsky pr, 10-2-18
Leningrad, 192241(SU)

Applicant: KUZNETSOVA, Valentina Alexeevna
ul.Bely Kuna, 22-3-14
Leningrad, 192241(SU)

Applicant: ROZENBERG, Mark Eduardovich
Kondratievsky pr., 79-13
Leningrad, 195266(SU)

Applicant: DENISENKO, Petr Prokofievich
Kljuchevaya ul., 3-35
Leningrad, 195221(SU)

Applicant: RAK, Arthur Vasilevich
shosse Revoljutsii, 33-1-49
Leningrad, 195176(SU)

Applicant: ADAMIAN, Arnold Aramovich
1 Baltiisky per., 23/25-66
Moscow, 125315(SU)

Applicant: GILMUTDINOV, Il Garafeevich
ul. Mayakovskogo, 19-32
Kazan, 420012(SU)

Applicant: KILKO, Vitaly Alexandrovitch
Primorsky pr., 19-24
Leningrad, 197010(SU)

Applicant: REVSKOI, Andrei Konstantinovitch
Tallinskaya ul., 12-407
Moscow(SU)

Applicant: LISITSYN, Konstantin Mikhailovich
ul. Kibalchicha, 2-1-108
Moscow, 129164(SU)

(72) Inventor: SOROKIN, Anatoly Yakovlevich
Tikhoretsky pr, 10-2-18
Leningrad, 192241(SU)
Inventor: KUZNETSOVA, Valentina Alexeevna
ul.Bely Kuna, 22-3-14
Leningrad, 192241(SU)
Inventor: ROZENBERG, Mark Eduardovich
Kondratievsky pr., 79-13
Leningrad, 195266(SU)
Inventor: DENISENKO, Petr Prokofievich
Kljuchevaya ul., 3-35
Leningrad, 195221(SU)
Inventor: RAK, Arthur Vasilevich
shosse Revoljutsii, 33-1-49
Leningrad, 195176(SU)
Inventor: ADAMIAN, Arnold Aramovich
1 Baltiisky per., 23/25-66
Moscow, 125315(SU)
Inventor: GILMUTDINOV, Il Garafeevich
ul. Mayakovskogo, 19-32
Kazan, 420012(SU)
Inventor: KILKO, Vitaly Alexandrovitch
Primorsky pr., 19-24
Leningrad, 197010(SU)
Inventor: REVSKOI, Andrei Konstantinovitch
Tallinskaya ul., 12-407
Moscow(SU)
Inventor: LISITSYN, Konstantin Mikhailovich
ul. Kibalchicha, 2-1-108



Xerox Copy Centre

Moscow, 129164(SU)

(74) Representative: **Ablewhite, Alan James**
**MARKS & CLERK 57/60 Lincoln's Inn Fields**
**London WC2A 3LS(GB)**

## (54) COPOLYMERS OF VINYL ALCOHOL WITH VINYL ACETATE, CROSS-LINKED BY GLUTARIC DIALDEHYDE, METHOD OF OBTAINING THEM AND A PHRAMACEUTICAL PREPARATION BASED THEREON.

(57) Novel compounds - copolymers of vinyl alcohol with vinylacetate cross-linked by glutaric aldehyde have the following structural formula:

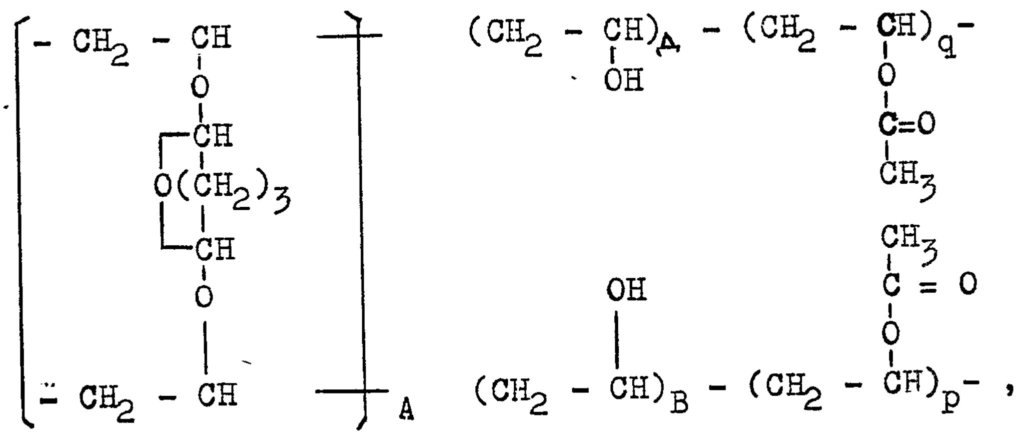

wherein

$$\frac{B+P+A+q}{A} = n = 30\text{-}125; \quad \frac{P+q}{B+A} = \ell \leq 0.2;$$

A = l, B + A = 30 - 125, A = 15 - 63, B = 15 - 63,
P + q = 0 - 25, q = 0 - 13, P = 0 - 13.

A method for preparing copolymers of vinyl alcohol with vinylacetate cross-linked by glutaric aldehyde which comprises mixing an aqueous solution of polyvinyl alcohol with a content of acetate groups of at most 20 mol. % with glutaric aldehyde at the ratio of 1,000 molar units of polyvinyl alcohol per 8-40 moles of glutaric aldehyde.

Said mixing is effected at a temperature of 15-80 °C under static conditions without stirring with a subsequent addition of a mineral acid to a pH = 1-3. The reaction mass formed as a hydrogel containing the desired product is disintegrated in a non-hydrating medium. A suspension is thus obtained, wherefrom disintegrated hydrogel is recovered and successively treated with water, a polar solvent and dried.

A pharmaceutical preparation exhibiting hemostatic, antiedemic, antiinflammatory and draining effects consists of an active principle - copolymers of vinyl alcohol with vinyl acetate cross-linked by glutaric aldehyde of the above-given structural formula.

# COPOLYMERS OF VINYL ALCOHOL WITH VINYLACETATE CROSS-LINKED BY GLUTARIC ALDEHYDE, METHOD FOR PREPARING SAME AND PHARMACEUTICAL PREPARATION BASED THEREON

## Field of the Invention

The present invention relates to organic chemistry and, more specifically, to novel compounds - copolymers of vinyl alcohol with vinylacetate cross-linked by glutaric aldehyde, method for preparing same and pharmaceutical preparation based thereon.

These copolymers are useful in medicine as a polyfunctional wound-healing dressing agent.

## Prior Art

Known in the art are various wound-healing and dressing agents exhibiting a hemostatic, antiedematic, antiinflammatory, draining effects of protecting the wound against the external factors.

Thus, known in the art is a compound featuring a hemostatic effect and comprising a monoiron salt of polyacrylic acid (preparation "Feracryl", SU, A, 698622). This preparation, when administered to patients, creates a higher risk for persons suffering from thrombosis by promoting the development of purulent processes in a wound (necessitates sterile conditions for its administration).

Known in the art is also biological antiseptic tampon exhibiting a hemostatic effect which comprises a composition of native proteins of blood plasma, gelatine, calcium chloride and sodium salt of benzylpenicillin. When administered to patients, this preparation can cause an antigenous response, creates the risk of thrombosis and infectioning of the wound (Alexandrova N.M. "Opportunities for a delayed treatment of wounds with BAT", Vestnik Khirurgii, No. 11, 1955, pp. 91-95).

Known is also a preparation "Dezhizan" (Germed company, GDR, wound-treatment preparation "Dezhizan") having antiedemic and anti-inflammatory effects which comprises a powder-like composition of regenerated cellulose, carboxymethylcellulose and polyethylene glycol. "Dezhizan" is capable of absorbing only 4-7 ml of water per gram of the preparation, thus necessitating a high rate of consumption of the preparation - - 250 mg/cm$^2$ and frequent wound dressings - 1-2 a day and, furthermore, application of a hemostatic agent when it is necessary to arrest a capillary hemorrhage.

It is also known to use a powder of polyvinyl alcohol with a particle size of 0.05-0.4 mm as an agent for the treatment of injuries of a different etiology (EP 0022064). The use of this preparation necessitates frequent wound dressings - 1-2 times a day. The bandage is removed as a crust, thus bringing about traumatization of the wound; moreover, the crust formation hinders water- and gas-exchange in the wound. The agent does not possess hemostatic and drainage properties.

Also known in the art is a dressing agent (EP 0097846) based on a cross-linked copolymer of vinyl alcohol and vinylformate combined with polyhydric $C_2$-$C_6$-alcohols in an amount of from 30 to 80% by weight of the copolymer. This preparation is capable of absorbing 6-8 g of water per 1 g of the agent,but it is impossible to use in the case of traumata (wounds) of joints, groin and other places inconvenient for dressing; furthermore, it does not ensure a required water- and air-exchange in the wound which might result in recurrence of inflammation, it neither has a hemostatic effect.

Known in the art is a method for cross-linking of polyvinyl alcohol with glutaric aldehyde (Colloid and Polymer Science, v.258, No.7, 1980, July; D.Braun und E.Walter "Intra- und Intermolekulare Vernetzung von Polivinylalkohol mit Dialdehyden") which comprises addition, to an aqueous solution of polyvinyl alcohol under an inert gas and stirring, of a 25% aqueous solution of glutaric aldehyde at a ratio of 1,000 molar units of polyvinyl alcohol per 0.38-3.08 mol of glutaric aldehyde and the pH value of the mixture is brought to 1-3 by adding hydrochloric acid to form a mass containing the product of reaction of polyvinyl alcohol with glutaric aldehyde. The desired products is recovered by way of precipitation of the polymer from the aqueous solution with methanol. However, as it is well known (EP,0097846), such products are non-elastic, rigid and cannot be used for wound-healing as a film-like dressing material.

Thus all the above-mentioned preparations don't possess the whole range of properties needed for wound--healing. They are characterized by a restricted range of action and side effects.

The claimed preparations are novel and are not described in the literature.

Summary of the Invention

It is an object of the present invention to provide a process for preparing novel compounds which would feature hemostatic antiedematic and antiinflammatory effects and capable of protecting the wound against an external infectioning and ensuring a painless dressing.

This object is accomplished by that, according to the present invention, novel copolymers of vinyl alcohol with vinylacetate cross-linked by glutaric aldehyde are proposed which have the following general structural formula:

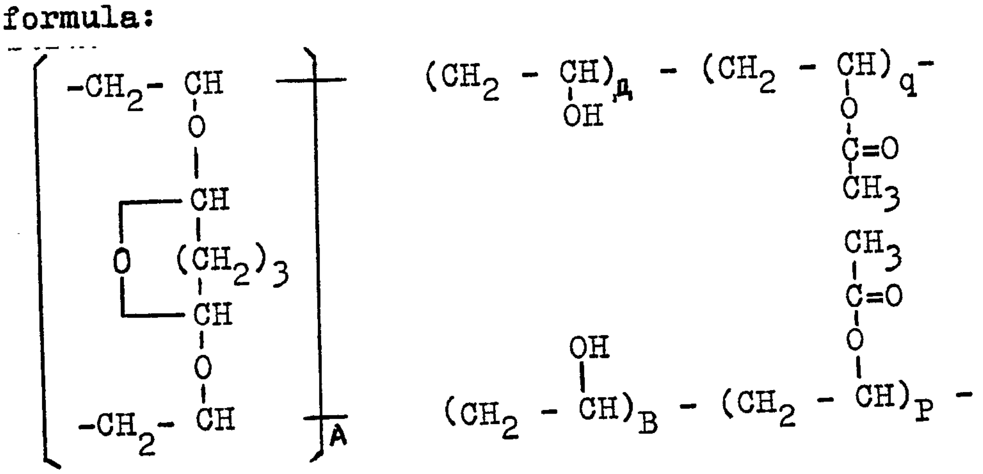

wherein:

$$\frac{B + P + Д + q}{A} = n = 30 \div 125; \quad \frac{P + q}{B + Д} = \ell \leq 0.2$$

A = 1

B + . = 30-125, Д = 15-63 B = 15-63,

P + q = 0-25, q = 0-13, P = 0-13.

The compounds according to the present invention comprise a powder-like substances which is a copolymer of vinyl alcohol with vinylacetate chemically cross-linked by means of glutaric aldehyde, insoluble in none of solvents, rapidly swelling in water and in dimethylsulphoxide or in a mixture of both.

The structure of the copolymers is proven by chemical analysis, IR-spectroscopy data containing bands of 1,050-1,200 $cm^{-1}$ (stretching vibrations of -C-O- groups in ethers and alcohols), 2,924 $cm^{-1}$ (stretching vibrations of -CH- groups); 2,855 $cm^{-1}$ (stretching vibrations of a sequence of $-CH_2$-groups).

The copolymers exhibit a strong hemostatic effect, antiinflammatory and entiedematic effects, a draining effect; they have a great sorption capacity (water-absorption within the range of from 700 to 2,100%) and a high rate of swelling in water. The time of maximum swelling in water and in a physiological solution does not exceed 10 minutes. At the same time, upon swelling in tissue fluids the copolymers do not cause necrosis of healthy cells of the soft tissues upon draining of wounds which points to the values of osmotic forces close to those of water in the copolymers according to the present invention and in normal cells of the tissues.

The preparation according to the present invention can be used independently and in combination with other preparations, intended to stimulate wound-healing processes, such as antiseptics, antibiotics and the like.

A method for preparing copolymers of vinyl alcohol with vinylacetate cross-linked by glutaric aldehyde consists in that an aqueous solution of polyvinyl alcohol with a content of acetate groups of not more than 20 mol.% is intermixed with glutaric aldehyde at a temperature within the range of from 15 to 80°C followed by the addition of mineral acid to a pH of from 1 to 3 with the formation of a reaction mass, wherein according to the present invention, the starting components are employed at a ratio of 1,000 molar units of polyvinyl alcohol per 8-40 moles of glutaric aldehyde; the cross-linking is effected under static conditions without stirring, the obtained reaction mass in the form of a hydrogel is disintegrated in a non-dehydrating medium, the resulting disintegrated hydrogel is recovered from the suspention obtained and successively treated with water and polar solvents and then dried.

As polyvinyl alcohol use is made of a high-molecular polymer containing up to 20 mol.% of acetate groups, preferably polyvinyl alcohol with a mean molecular mass ranging from 50,000 to 130,000.

The disintegration of the hydrogel of the desired product is effected in a non-dehydrating medium. As it is known, the non-dehydrating medium is a medium which does not take water from the treated product. Such medium can be exemplified by air, water, a mixture of a polar organic solvent with water, polar and non-polar organic solvents (preferably water, mixtures water-acetone, water-dioxane, water-methanol, water-ethanol, more preferably water or mixtures water-acetone.

As the polar solvent for the treatment of the disintegrated hydrogel of the copolymers according to the present invention use can be made of ketones, ethers, alcohols, preferably acetone,dioxane, ethanol, methanol.

As the mineral acid use is made of sulphuric acid, hydrochloric acid, nitric acid, iodous acid, bromous

acid, sulphonic acids and the like, preferably hydrochloric and orthophosphoric acid.

The above-specified range of the process temperature and pH is optimal. Thus, at a temperature below 15°C the rate of the reaction of polyvinyl alcohol with glutaric aldehyde is low, while at a temperature of above 80°C the reaction rate is so high that to obtain a high-quality desired product a very high intensity of stirring of the reaction mass is required at the stage of intermixing of the components. As regards the pH of the reaction medium, at a pH of more than 3 the rate of the above-mentioned reaction is also very small, where as at a pH below 1 an excessive increase of the reaction speed is observed. The presence of a mineral acid in the reaction medium ensures a sufficient concentration of hydrogen ions therein to provide the required rate of the reaction between units of vinyl alcohol and glutaric aldehyde.

The selected ratio of the starting components, namely: 8-40 moles of glutaric aldehyde per 1,000 molar units of polyvinyl alcohol ensures the required structure of the copolymers of vinyl alcohol with vinylacetate cross-linked by glutaric aldehyde and the desired complex of their useful properties.

The use of the combination of a chemical cross-linking of polyvinyl alcohol in an aqueous solution under static conditions, followed by disintegration of the resulting hydrogel in a non-dehydrating medium, successive treatment with water and a polar solvent with the subsequent drying ensures the production of the copolymers of vinyl alcohol and vinylacetate cross-linked by glutaric aldehyde which are harmless for a human organism and which possess a new range of therapeutic properties.

The compounds prepared by the process according to the present invention have a hemostatic effect, antiedematic, antiinflammatory and draining activity, and are acting compounds of the pharmaceutical preparation.

Best Mode to Carry out the Invention

The copolymers according to the present inventon have been subjected to experimental and clinical tests.

The local irritating effect of the copolymers of vinyl alcohol with vinylacetate cross-linked by glutaric aldehyde was studied on 12 white male rats and on 6 rabbits in the following manner.

Thoroughly washed tails of the rats from the test group were placed into test-tubes with a 10% suspension of the copolymer according to the present invention in water, while for the control group - into test-tubes with distilled water for 4 hours daily over the period of 12 days; in so doing, in 50% of the animals of each group 3-4 notches were made on the tails by means of a razor blade. At the same time, the copolymer according to the present invention was applied onto a shaved region of the skin on the right side of the same rats.

The daily observations over the rats have shown that no changes in the skin coats in the animals of the test group are observed as compared to the rats of the control group. No deviations from the norm were also observed in the behaviour of the animals.

The test rabbits were daily, over the period of 12 days, subjected to administration of a 10% suspension of the copolymer behind the lower eyelid of the right eye and dropping of distilled water into the left eye.

No changes in the mucous tissues of the rabbits' eyes upon the treatment with the copolymer were observed.

Therefore, the copolymer according to the present invention provides no irritation effect on the skin and

mucous tissues.

The study of allergenic properties of the copolymer of the present invention was effected by the method of lysis of leucocytes in vitro and by the method of specific agglomeration of leucocytes. The experiments were carried out on 13 male guinea pigs (6 animals in a group) with a mass of 300 to 350 g by way of a complex administration of the copolymer according to the present invention and a physiological solution into the ear's skin. Then, after 10 days during the following 7 days daily apicutant applications were made. To the animals of the 1-st test group the copolymer was administered in the dilution of 1:500, to the animals of the 2-nd group - in the dilution of 1:1,000; the animals of the 3-rd (control) group were administered with a physiological solution. The results of the experiments are shown in Tables 1 and 2 hereinbelow.

The results of the experiments (the analysis and comparison of the results were effected by the statistical method based on the Student criterion) have shown that certain differences are absent between all groups of the animals (the control and test ones).

Consequently, the copolymer according to the present invention provides no allergenic effect.

The study of the general toxic effect of novel compounds according to the present invention was effected on 115 white rats which were administered hypodermally in the back region, under hexanal narcosis, with the test compound in the dose of 1 g per kg of the animal's bodyweight. All the animals were divided into three groups: 1 - control (pseudo-operated); II - the animals to which the copolymer was grafted hypodermally; III - talcum was hypodermally introduced.

The animals of each group were tested for:

-morphological composition of the blood;

Table 1

Reaction of lysis of leucocytes

| Group of animals | Number of leucocytes, $10^9$ pieces/litre | | |
|---|---|---|---|
| | Prior to experiment | 10 days after intradermal injection | |
| | | without the compound | with the compound |
| 1 | 2 | 3 | 4 |
| Group 1 (dilution 1:500) | 19.2±0.90 | 19.0±0.9 | 16.6±0.84 |
| Group 2 (dilution 1:1,000) | 16.8±0.86 | 16.7±0.86 | 16.0±0.99 |
| Group 3 (dontrol) | 18.0±0.82 | 18.0±0.87 | 17.7±0.83 |

Table 1 (continued)

| Group of animals | Number of leucocytes, $10^9$ pieces/litre | |
|---|---|---|
| | After 7 daily skin applications | |
| | without the compound | with the compound |
| 1 | 5 | 6 |
| 1 | 18.9±0.95 | 17.1±0.83 |
| 2 | 16.6±0.92 | 15.8±0.97 |
| 3 | 18.0±0.77 | 17.8±0.89 |

Table 2

Reaction of specific agglomeration of leucocytes

| Group of animals | Number of agglomerated leucocytes,% (points) | |
|---|---|---|
| | Prior to experiment | 10 days after intradermal administration |
| 1 | 2 | 3 |
| Group I (dilution 1:500) | 12.3 (0) | 13.7(0) |
| Group 2 (dilution 1:1,000) | 10.3 (0) | 10.6 (0) |
| Group 3 (control) | 11.0 (0) | 12.1 (0) |

Table 2 (continued)

| Group of animals | Number of agglomerated leucocytes,% (points) |
|---|---|
| | After 7 daily skin applications |
| 1 | 4 |
| 1 | 12.4 (0) |
| 2 | 11.8 (0) |
| 3 | 10.8 (0) |

- biochemical characteristics of the blood showing the liver function on the 15-th and 30-th day after the operation;
- function of kidneys according to the dynamics of diuresis and urina analysis on the 15-th and 30-th day after the operation;
- function of the central nervous system by the method of the summary-threshold indicator (packets of pulses of 200 microseconds were used with elevation of the voltage amplitude from 1 to 20 V to evaluate responses to electric current) on the 15-th and 30-th day after the operation;
- dynamics of the animals' mass on the 10-th, 20-th and 30-th day after the operation and weight coefficients of the inner organs (heart; lungs, liver, kidneys, adrenal gland spleen).

The analysis and comparison of the results of

these studies carried ou. with the use of the Student criterion have shown that certain differences are absent between all the groups of animals.

30 days after the grafting of the test compound under the skin of the rats the animals were slaughtered and histomorphological investigations of the inner organs (lungs, liver, kidneys, heart, spleen, adrenal glands) and the skin were performed. The results of these investigations have shown that the copolymer causes no pathological changes in the inner organs and the skin of the animals.

Therefore, the study of the general toxic effect of the novel copolymers shows that they are harmless for a live organism.

The study of the response of tissues to the copolymers according to the present invention was effected on 120 white rats and 27 male rabbits. The wounds were made in the back under hexanal narcosis by removing a piece of the skin of 10 mm diameter in rats and of 30 mm diameter in rabbits. In each series of the experiments the animals were divided into three groups: Group I - wounds without treatment (control); 2 - wounds are poured with talcum; 3 - wounds are poured with the copolymer of the invention. The crust was removed daily from the rats of all the groups, in rabbits of all of the groups the crust was removed every second day. In all cases the wound area was measured and the test substance was applied again.

During the process of wound healing histomorphological studies of the wound defect were performed in dynamics on the 1-st, 4-th, 7-th, 10-11-th, and 15-16-th day after wounding.

On the 3-rd day a purulent excretion was observed: in the non-treated animals - in 88% of them, in talcum-treated animals - in 60%, and in copolymer-treated

animals - in 40% of them.

The highest speed of decreasing of the wound surface areas was observed in the case of using the novel copolymer. A full healing of the wounds took 40 days in rats, 40 days in rabbits, whereas in the non-treated rats this time was 60 days, rabbits - 59 days; for the talcum-treated animals this was 65 days for rats and 60 days for rabbits.

The data of the histomorphological studies have shown that the copolymers do not hinder the development of regeneration and reparation processes in the wound.

Therefore, the application of the novel compounds onto the wound not only has no harmful effect, but also accelerates the wound healing.

To evaluate the hemostatic effect the new polymer was used which had n = 67, l not more than 0.001.

The experiment was conducted on 2 groups of white rats with a mass of 170-250 g and having 6 animals in each.

The animals were subjected to a partial hepatectomy (1/4-1/3 part of the liver was cut-off) under a local narcosis and then the test substances were applied onto the bleeding surface.Thereafter, the time of arresting of the parenchymatous bleeding was measured.

In the group of animals treated with the copolymer the bleeding was stopped after 12.2±1.2 sec, whereas in the group of animals treated with a biological antiseptic tampon - within 36.0±5.5 sec.

For evalutation of the hemostatic effect use was also made of the copolymer according to the present invention with n = 30 and l = 0.005. The experiment was conducted on 2 groups of non-descript dogs containing 6 animals each.

The dogs were subjected to wounding of the skull and brain under a general barbiturate narcosis.

In the group treated with the novel copolymer

the capillary bleeding hemostasis was observed within 5-7 seconds, whereas in the group treated with a biological antiseptic tampon the hemostatic effect was observed within 17-25 sec.

To evaluate the hemostatic effect use was also made of a powder of the copolymer with n = 125 and 1 = at least 0.001.

The experiment was effected on 2 groups of nondescript dogs with 10 animals in each group. The dogs were subjected to an open penetrating wounding of the skull and brain under barbiturate narcosis.

In the group treated with the copolymer the capillary bleeding was stopped after $3\pm0.8$ sec. The reduction of the number of erythrocytes relative to the initial level was $(0.9\pm0.17)\times10^9$ pieces/$cm^3$.

In the group treated with the biological antiseptic tampon the hemostatic effect was observed within $60\pm14$ sec. The reduction of the number of erythrocytes relative to the initial level was $(1.7\pm0.32)\times10^9$ pieces/$cm^3$.

The total loss of blood determined by the animals' mass in the case of administration of the copolymer was by $1.8\pm0.2$ times lower in comparison with the case of utilization of the biological antiseptic tampon.

In all the animals treated with the copolymer there was noted visually the reduction of the brain volume (diminution of edema) which was revealed in a clear retraction in the zone of the trepanation defect, appearance of an active pulsation of the brain. Morphological studies of the brain tissues have shown that the new copolymers do not increase the zones of a secondary necrosis in the wound.

To evaluate the effect of the novel compounds on the blood coagulation system use was made of a powder of the copolymer with n = 83 and 1 = 0.005.

The experiment was conducted on 2 groups of rabbits with a mass of 3-3.5 kg, 5 animals of each group. A skin piece of 30 mm diameter was removed in the test animals. In the rabbits of the test group a powder of the copolymer was applied onto the wound, whereafter blood was taken from the animals of the test and control groups within various time limits and subjected to examination in a thromboelastograph. The results of the analysis are shown in Table 3 hereinbelow.

Table 3

| Group | Parameter of the hemostatic effect | Prior to the experiment | Time after making the trauma, days | | |
|---|---|---|---|---|---|
| | | | 10 | 20 | 30 |
| Test group (treated with the copolymer) | P | 16.5±0.9 | 21.5±9.4 | 17.5±4.4 | 14.0±3.5 |
| | K | 12.5±0.9 | 20.5±8.8 | 18.0±3.5 | 14.0±0.9 |
| | MA | 77.0±3.5 | 80 ±8.5 | 80.5±6.2 | 73.0±5.3 |
| Control | P | 16.0±1.8 | 14.0±8.8 | 10.5±I.8 | 11.0±0.8 |
| | K | 12.5±0.8 | 15.0±7.1 | 14.0±7.1 | 12.1±0.9 |
| | MA | 72.5±8.0 | 82.0±3.5 | 76.5±8.0 | 72.5±0.9 |

wherein:

P - thromboelastographic constant proportional to the time of recalcification of the plasma and the time of coagulation;

K - thromboelastographic constant of thrombin characterizing the speed of blood coagulation and formation of a clot;

MA- maximum amplitude where the clot reaches the maximum volume, density and elasticity.

The statistical processing of the results and comparison of the data for the test and control groups carried out with the aid of the Student criterion demonstrate that in the control group the coagulation of the

peripheral blood of the animals has increased, whereas in the group treated with the novel preparation it not only showed no increase, but even was slightly lowered.

For evaluation of the hemostatic effect use is made of a powder of a copolymer of vinyl alcohol with vinylacetate cross-linked with glutaric aldehyde, n = = 125, l = 0.176.

The experiment was carried out on white non-descript rats with a mass of 170-250 g, 6 animals in each group. The rats were subjected to a partial hepatectomy (1/4-1/3) portion of the liver was cut-off) under a local narcosis and a hemostatic agent was applied onto the bleeding surface, whereafter the time of hemostasis was measured.

After application of the hemostatic agent the bleeding arrested in the group of animals treated with the copolymer after 12.6±1.8 sec., in the group treated with the biological antiseptic tampon - after 33+4.7 sec.

The clinical assessment of the hemostatic effect of the copolymer according to the present invention with n = 83 and l = 0.005 was effected in two groups of patients of 5 persons in each operated on the occasion of the removal of tumors in lungs and thoracal wall, for hemostasis in the subcutaneous-fat and muscular layers at a capillary hemorrhage.

In the group treated with the copolymer according to the present invention the hemostatic effect was observed within 25-40 seconds; in the group treated with a biological antiseptic tampon - within 60-80 seconds.

For assessment of the hemostatic effect the copolymer according to the present invention with n = = 71, l = 0.104 was used.

Clinical tests were carried out in two groups of patients of 4 persons in each operated on the occasion of the removal of Madelung lipoma for hemostasis in the subcutaneous fat layer at a capillary hemorrhage.

In the group treated with the copolymer according to the present invention the hemostatic effect was observed within 25-38 seconds, while in the group treated with a biological antiseptic tampon - within 60-80 seconds.

For assessment of the hemostatic effect the copolymer according to the present invention with n = 80 and 1 = of not more than 0.001 was used. Clinical tests were carried out in two groups of patients of 6 persons in each operated on the occasion of chronic hematogenic and post-operational osteomielitis of femur and crus, for hemostasis in the skin-fat layer at a capillary hemorrhage.

In the patients treated with the copolymer according to the present invention the hemorrhage was stopped within 25-40 seconds, in the group treated with a biological antiseptic tampon - within 60-100 seconds.

The copolymer of vinyl alcohol and divinyl glutarate according to the present invention was studied in clinic. Clinical tests of the copolymer according to the present invention were carried out on 500 patients with different purulent inflammatory diseases and complications.

The tests were conducted with patients aged 15 to 80 years of both sexes and with different localization of wounds.

In the test group the patients were divided into two subgroups:

- acute purulent-inflammatory diseases of soft tissues (abscess, phlegmon, furuncle, carbuncle, mastitis, post-operational and post-traumatic purulent

wounds, allergic and microbal skin necrosis);

- chronic purulent-inflammatroy diseases of soft tissues (trophic ulcers, osteomyelitis, post-operational and post-traumatic wounds with a long period of healing, bedsores, pyothorax).

At the same time, in the control group of 200 patients groupped in a manner similar to that of the test group the treatment was effected using conventional pharmaceutical preparations administered in the first stage of the wound process (10% aqueous solution of sodium chloride, chlorophylite, microcyte, aqueous solutions of dioxidine, chlorohexidine and furacin).

The clinical tests were aimed at:

- elaboration of indications and modes of treatment with the compolymer according to the present invention;

- assessment of the efficiency of the copolymer according to the present invention.

The treatment by the copolymer of vinyl alcohol and divinylglutarate was carried out in the following manner.

When necessary, the purulent-inflammatroy focus was subjected to a surgical treatment, whereafter the wound surface was subjected to hemostasis, followed by toilet of the wound (drying with tampons, washing with a 3% solution of hydrogen peroxide and a repeated drying). The copolymer according to the present invention was powdered on the wound. In the case of abundantly secreting wounds the copolymer was applied in the amount of 20 mg/cm$^2$, at a moderate secreting 6 to 10 mg/cm$^2$. The wound was covered with a dry aseptic dressing. In the case of abundantly secreting wounds such dressings were effected daily, at a moderately secreting wound - once during 2-5 days till a full cleaning of the wounds from pus and necrotized tissues

and till the appearance of a granulation tissue. In each dressing the copolymer saturated with the exsudate was removed during the toilet of the wound by washing it off with a jet of an antiseptic solution, followed by drying with a tampon. Taking into consideration the hemostatic effect of the copolymer according to the present invention no other hemostatic preparations were used during the treatment with the preparation as claimed. In the control group for hemostatic purposes use was made of trombin, hemostatic sponge, biological antiseptic tampon.

During the clinical observation of the progress of the 1-st stage of the wound process in patients with acute purulent-inflammatory processes in soft tissues in the case of treatment with the copolymer according to the present invention there was noted a reduction, by about 2 times, of the duration of elimination of edema, infiltration and hyperemia of the underlying tissues (Table 4). The patients of the test group found that already on the first day of the treatment the pain feelings were considerably diminished in contrast to the patients of the control group.

A full cleaning of the wound from purulent-necrotic mass and appearance of granulations in the patients of the test group occurred within approximately the same time limits by 1.5-2 times earlier than in the patients of the control group.

Bacteriological studies of the level of infectioning of wounds in the above-mentioned patients have shown that in the case of treatment with the copolymer according to the present invention, in contrast to the control group, already on the 3-4-th day the number of microbal bodies was not higher than $10^3$ bacteria per 1 $cm^2$, while on the 7-8-th day the growth of microorganisms was not observed (Table 5). The dynamics

of a cytological pattern of the wound process (Table 6) also demonstrated a considerable acceleration of its 1-st phase whose duration for the copolymer according to the present invention was 7-8 days, whereas in the control it was equal to 11-12 days.

Clinical observations of the wound process of chronic purulent-inflammatory diseases of soft tissues and bones (trophic ulcers, osteomielitis, post-operational and post-traumatic wounds with a long period of healing, bedsores, pyothorax) showed reduction of the healing duration by about 2.5 times as compared to the control group, as well as the duration of elimination of edema, inflitration and hyperemia of the underlying tissues (see Table 4).

Table 4

Clinical characteristics of the progress of the 1-st phase of the wound process

| Character of purulent-inflammatory process | Group of patients | Number of patients in the group | Time of elimination of edema, days |
|---|---|---|---|
| 1 | 2 | 3 | 4 |
| Acute | Test | 300 | 2.3±0.2 |
| | Control | 150 | 4.1±0.3 |
| Chronic | Test | 200 | 4.3±0.5 |
| | Control | 50 | 10.0±1.0 |

Table 4 (continued)

| Time of elimination of inflammatory reaction (hyperemia and inflitration of underlying tissues), days | Time of cleaning of the wound from purulent-necrotic masses, days | Time of appearance of granulation tissue, days |
|---|---|---|
| 5 | 6 | 7 |
| 2.8±0.4 | 4.9±0.5 | 5.2±0.5 |
| 6.1±0.5 | 8.7±0.7 | 11.5±0.8 |
| 6.9±0.8 | 10.5±0.8 | 11.3±1.5 |
| 14.3±1.2 | 19.7±2.3 | 22.3±2.7 |

Table 5

Dynamism of the level of infectioning of wounds during the treatment x)

| Character of purulent-inflammatory process | Groups of patients | Number of patients in the group | Number of microbal bodies per 1 $cm^2$ of the wound surface | |
|---|---|---|---|---|
| | | | Prior to the treatment | On the 3-4-th day |
| 1 | 2 | 3 | 4 | 5 |
| Acute | Test | 50 | $10^5$-$10^7$ | $10^2$-$10^3$ |
| | Control | 20 | $10^5$-$10^7$ | $10^4$-$10^5$ |
| Chronic | Test | 50 | $10^5$-$10^7$ | $10^3$-$10^4$ |
| | Control | 20 | $10^5$-$10^7$ | $10^5$-$10^7$ |

x) In the bacteriological control the following varieties of microflora were inoculated: staphylococcus aureus, blue pus bacillus, proteus, colibacillus. These bacteria, as a rule, encountered in associations.

Table 5 (continued)

| Character of purulent-inflammatory process | Groups of patients | Number of patients in the group | Number of microbal bodies per 1 $cm^2$ of the wound surface | |
|---|---|---|---|---|
| | | | 7-8-th day | 11-12-th day |
| 1 | 2 | 3 | 6 | 7 |
| Acute | Test | 50 | No growth | - |
| | Control | 20 | $10^2$-$10^3$ | No growth |
| Chronic | Test | 50 | $10^1$-$10^2$ | No growth |
| | Control | 20 | $10^4$-$10^5$ | $10^3$-$10^4$ |

Table 5 (continued)

| Character of purulent-in-flammatory process | Groups of patients | Number of patients in the group | Number of microbal bodies per 1 cm of the wound surface | |
|---|---|---|---|---|
| | | | 15-16-th day | 19-20-th day |
| 1 | 2 | 3 | 8 | 9 |
| Acute | Test | 50 | - | - |
| | Control | 20 | - | - |
| Chronic | Test | 50 | - | - |
| | Control | 20 | $10^2$-$10^3$ | $10^1$ |

In this case, likewise in the group of patients with an acute purulent-inflammatory process, in the patients of the instant group reduction of pain feelings and itching was noted during the first two days.

A full cleaning of the wound from purulent-necrotic masses and appearance of granulations in the patients of the test group occurred approximately within the same time limits but by 1.5-2 times faster than in the patients of the control group.

Bacteriological studies of the level of infection-

Table 6

Dynamics of the cytological pattern during treatment of wounds

| Character of purulent-inflammatory process | Groups of patients | Number of patients in the group | Type of cytogram | | |
|---|---|---|---|---|---|
| | | | Prior to treatment | On the 3-4-th day | On the 7-8-th day |
| 1 | 2 | 3 | 4 | 5 | 6 |
| Acute | Test | 50 | Inflammatory-degenerative | Inflammatory | Regenerative |
| Acute | Control | 20 | Inflammatory-degenerative | Inflammatory-regenerative | Inflammatory-regenerative |
| Chronic | Test | 50 | Inflammatory-degenerative | Inflammatory | Inflammatory |
| | Control | 20 | Inflammatory-degenerative | Inflammatory-degenerative | Inflammatory |

Table 6 (continued)

| Character of purulent-inflammatory process | Groups of patients | Number of patients in the group | Type of cytogram | | |
|---|---|---|---|---|---|
| | | | 11-12-th day | 15-16-th day | 19-20th day |
| 1 | 2 | 3 | 7 | 8 | 9 |
| Acute | Test | 50 | *) | - | - |
| | Control | 20 | Regenerative | *) | - |
| Chronic | Test | 50 | Inflammatory-regenerative | Regenerative | *) |
| | Control | 20 | Inflammatory | Inflammatory | Inflammatory regenerative |

*) Cytological studies were not carried out, since the process of epithelization occurred or the wounds were closed by secondary sutures or autodermoplasty was effected.

ing of wounds in the above-mentioned patients showed that in the case of treatment with the copolymer according to the present invention, in contrast to the control group, already on the 3-4-th day the number of microbal bodies per 1 $cm^2$ of the wound surface did not exceed $10^4$ bacteria, on the 7-8-th day - not more than $10^2$ bacteria per 1 $cm^2$ of the wound surface, while on the 11-12-th day the growth of microorganisms was not observed (Table 5). The dynamics of the cytological pattern of the wound process (Table 6) also pointed to a considerable acceleration of its 1-st stage which for the copolymer according to the present invention was 15-16 days.

It should be noted that in wounds healed by the copolymer according to the present invention, in contrast to the control group, the species and spectrum of microflora had no influence on the speed of cleaning and healing of the wound which occurred within the shortest biologically acceptable time limits depending on severity of the purulent-inflammatory process.

In the test groups, unlike in the control one, in none of the patients a secondary infectioning of the wound was noted which demonstrated reliable protective properties of the hydrogel layer formed by the copolymer according to the present invention upon its swelling in the wound secretion.

It follows from the above-given results that, irrespective of etiology and localization of the wound, upon treatment with the copolymer according to the present invention the diminution of edema was accompanied by lowering of the intensity of pains in the zone of the purulent-necrotic focus or even by their full disappearance.

Along with the process of elimination of edema

the pronouncedness of the local inflammatory response - hyperemia of the ambient tissues was also reduced, likewise the phenomena of regional lymphadenitis and lymphangitis.

The acceleration of cleaning of wounds from purulent-necrotic masses and appearance of the granulation tissue in the treatment with the copolymer according to the present invention demonstrated a considerable activation of reparation processes and improvement of microcirculation in the wound.

Observations of the process of healing of wounds in the patients of the test group showed that the hydrogel layer formed upon application of the copolymer according to the present invention on the wound ensured an optimal water and air exchange.

The copolymer according to the present invention was well tolerated by the patients; it provided no allergic or toxic effect, caused no secondary necrose. No complications associated with its application were observed.

No contraindications against the use of the copolymer according to the present invention were found.

Therefore, the clinical investigation of the copolymer according to the present invention has shown that it exhibits a whole range of properties: it is a powerful hemostatic agent has an antiedemic and an antiinflammatory effect, accelerates cleaning of wounds from purulent-necrotic masses by sorbing decay products and microbial bodies, lowers the level of infectioning of the wound, protects the latter from outside infectioning, ensures a good gas- and water exchange in the wound and efficiently improves microcirculation therein.

When the copolymer according to the present invention is applied onto the bleeding wound surface, it

stops bleeding so that the coagulation ability of the peripheral blood is slightly lowered rather than increased which points to decreasing of the risk of thrombogenesis in patients liable to thrombosis.

The use of the copolymer according to the present invention, owing to its polyfunctional character, ensures both the hemostatic effect and therapeutic effect at the same time thus minimizing the number of pharmaceutical preparations employed in the treatment of purulent wounds, reduces the number of the required manipulations, the number of dressings and shortening the time of treatment.

In contrast to conventionally employed agents, in the treatment with the copolymer according to the present invention the removal of a bandage is painless and does not cause a secondary traumatization of the wound. Patients treated with the copolymer according to the present invention felt no discomfort or psychological pressure during dressings.

Clinical tests have shown that the copolymer of this invention reduces both the time of healing and forms considerably more tender, elastic and mobile scars.

The process for preparing copolymers of vinyl alcohol with vinylacetate cross-linked with the aid of glutaric aldehyde is effected in the following manner.

An aqueous solution of polyvinyl alcohol is prepared in a reactor in a conventional manner, whereafter charged into the reactor is the required amount of the aqueous solution of glutaric aldehyde, stirred, the mixture is heated to the reaction temperature and, by adding a mineral acid to the resulting solution, the reaction mass pH is brought to 1-3. As a result of the process of a chemical cross-linking under static conditions without stirring, the solution of the starting components of the reaction turns into a hydrogel of a copolymer of vinyl alcohol with glutaric aldehyde. The hydrogel is disintegrated in a non-dehydrating medium by means of a rotating impeller of the reactor to give a suspension of the hydrogel. The disintegrated hydrogel is recovered from the suspension, washed with water in the reactor to a pH of 6-7, whereafter it is treated with a polar solvent and dried.

For washing and drying of the desired product use is made of conventional techniques employed for the treatment of suspensions.

For implementation of the process according to the present invention use can be made of standard process equipment employed in the chemical industry (reactors, filters, centrifuges, pumps, heat-exchangers, driers and the like).

The production capacity and time characteristics of the process are defined by the appareillage employed in the process for producing the copolymers, by the level of automation and additional requirements imposed on the desired product.

For a better understanding of the present invention, some specific examples are given hereinbelow by way of illustration.

Example 1

200 g of an aqueous solution of polyvinyl alcohol containing 10 g of the polymer with the molecular mass of 90,000 with the content of acetate groups of 0.1 mol %, are placed into a 300 ml vessel, added with 1.41 g of a 25% aqueous solution of glutaric aldehyde. The ratio of molar units of polyvinyl alcohol to the number of moles of glutaric aldehyde is 1.000:15.5. Then the mixture is heated to the temperature of 60°C and added with hydrochloric acid to the pH of the reaction medium of 1.5. The resulting reaction mass is

stirred and allowed to stand for 4 hours. The thus-obtained hydrogel is placed into a disintegrator, added with 350 g of water and disintegrated. The disintegrated hydrogel is recovered from the suspension by filtration, washed with water, again filtered and treated with acetone, then again filtered and dried.

To improve purity of the desired product, the washing of the hydrogel of the obtained product can be repeated twice.

The properties of the resulting product and characteristics of its structure are shown in Table 7 hereinbelow.

Example 2.

The synthesis is effected following the procedure described in Example 1 hereinabove, except that polymer with the molecular mass of 90.000 with the content of acetate groups of 0.5% mol. and the ratio of molar units of polyvinyl alcohol to the number of moles of glutaric aldehyde is 1,000:40, the reaction temperature is 15°C, the non-dehydrating medium is air.

The properties of the resulting product and characteristics of its structure are shown in Table 7 hereinbelow.

Example 3

The synthesis is carried out following the procedure described in Example 1, except that the employed polyvinyl alcohol has the content of acetate groups of 18 mol.%, the ratio of molar units of polyvinyl alcohol to the number of moles of glutaric aldehyde is 1,000:8, the reaction temperature is 80°C, the non-dehydrating medium is a mixture of 210 g of water and 140 g of acetone.

The properties of the resulting product and characteristics of its structure are shown in Table 7 hereinbelow.

Example 4

The synthesis is carried out following the procedure described in Example 1, except that the employed polyvinyl alcohol has the molecular mass of 50,000 and the content of acetate groups of 3.3 mol.%; the ratio of molar units of polyvinyl alcohol to the number of moles of glutaric aldehyde is 1,000:37.5, the reaction temperature is 15°C, the non-dehydrating medium is a mixture of 175 g of water and 175 g of ethanol, the polar solvent - ethanol.

The properties of the thus-obtained product and characteristics of its structure are shown in Table 7 hereinbelow.

Example 5

The synthesis is carried out following a procedure similar to that described in Example 1 hereinbefore, except that the employed polyvinyl alcohol has the molecular mass of 130,000, the content of acetate groups of 0.8 mol.%, the ratio of molar units of polyvinyl alcohol to the number of moles of glutaric aldehyde is 1,000:8.5, the reaction temperature is 60°C, the non-dehydrating medium is a mixture of 175 g of water and 175 g of ethanol, the polar solvent is ethanol.

The properties of the desired product and characteristic of its structure are shown in Table 7 hereinbelow.

Example 6

The synthesis is effected following the procedure described in Example 1, except that the ratio of molar units of polyvinyl alcohol to the number of moles of glutaric aldehyde is 1,000:14.5, the temperature of the reaction mixture is 50°C, the non-dehydrating medium is a mixture of 120 g of water and 230 g of ethanol, the polar solvent is ethanol.

The properties of the resulting product and characteristics of its structure are shown in Table 7

hereinbelow.

Example 7

The synthesis is carried out following the procedure described in Example 1 hereinbefore, except that the employed polyvinyl alcohol has the content of acetate groups of 18.0 mol.%, the ratio of molar units of polyvinyl alcohol to the number of moles of glutaric aldehyde is 1,000:15.0, the reaction temperature is 50°C, the non-dehydrating medium is a mixture of 140 g of water and 210 g of acetone, the polar solvent is methanol.

The properties of the obtained product and characteristics of its structure are shown in Table 7 hereinbelow.

Example 8

The synthesis is conducted following the procedure described in Example 1, except that the employed polyvinyl alcohol has the molecular mass of 50,000, the ratio of molar units of polyvinyl alcohol to the number of moles of glutaric aldehyde is 1,000:19.5, the reaction temperature is 30°C, the non-dehydrating medium is a mixture of 230 g of water and 120 g of acetone, the polar solvent is dioxane.

The properties of the resulting product and characteristics of its structure are shown in Table 7 hereinbelow.

Example 9

The synthesis is effected following the procedure described in Example 1, except that the employed polyvinyl alcohol has the molecular mass of 130,000, the ratio of molar units of polyvinyl alcohol to the number of moles of glutaric aldehyde is 1,000:8.5, the reaction temperature is 55°C, the non-dehydrating medium is a mixture of 140 g of water and 120 g of ethanol.

The characteristics of the resulting product and those of its structure are shown in Table 7 hereinbelow.

Example 10

The synthesis is effected following the procedure described in Example 1, except that the employed polyvinyl alcohol has the content of acetate groups of 20 mol.%, the ratio of molar units of polyvinyl alcohol to the number of moles of glutaric aldehyde is 1,000:15.0, the reaction temperature is 25°C, the non-dehydrating medium is a mixture of 175 g of water and 175 g of dioxane, the polar solvent is dioxane.

The properties of the resulting product and characteristics of its structure are shown in Table 7 hereinbelow.

Example 11

The synthesis is effected following the procedure described in Example 1, except that the employed polyvinyl alcohol has the content of acetate groups of 3.3 mol.%, the ratio of molar units of polyvinyl alcohol to the number of moles of glutaric aldehyde is 1,000:37.5, the reaction temperature is 40°C, the non-dehydrating medium is a mixture of 175 g of water and 175 g of methanol, the polar solvent is methanol.

The properties of the resulting product and characteristics of its structure are shown in Table 7 hereinbelow.

Examples 12 and 13 illustrate the treatment of patients with the preparation according to the present invention in combination with other pharmaceutical preparations.

Example 12

Male patient M, aged 37, was treated on the occasion of chronic osteomyelitis of the right femur. The focus of osteomyelitis was surgically treated. A

A wound of 7x7x2 cm size was formed. On the surface of the purulent wound, after washing with a solution of hydrogen peroxide and furacin, the preparation was applied along with known preparations of chemotrypsin and carbenicillin disoidum salt. Dressing was effected once every three days. After 14 days the wound was fully filled with granulations. The patient was examined two years thereafter. Healthy.

Example 13

Female patient B, aged 39, was treated on the occasion of an acute purulent right-side mastitis. The abscess of the right mammal gland was opened by an arch-like incision of 7 cm length. After excretion of pus from the abscess cavity a wound of 100 $cm^3$ size (or 7x5x3 cm) was formed with its walls covered by a purulent-necrotic deposit with an abundant purulent secretion. After washing with a solution of hydrogen peroxide the preparation was applied onto the wound surface together with known preparations trypsin and dioxidine. The second dressing was made one day thereafter, the next dressing were performed once every three days. After 7 days the wound was fully cleaned from purulent-necrotic masses, the wound walls were covered with bright-red shady granulations. Sutures were applied onto the wound and 14 days thereafter it healed by the primary tension.

Industrial Applicability

Copolymers of vinyl alcohol with vinylacetate cross-linked by glataric aldehyde can find application in medicine for treatment of wound and burns of various etiology and localization, in particular for treatment of purulent complications and osteomyelites as hemostatic, antiedematic, antiinflammatory and draining preparation protecting tissues from external infection, accelerating cleaning of wounds from purulent necrotic masses and appearance of the granulation tissue.

Table 7

Characteristics of the properties and structure of copolymers of vinyl alcohol with vinylacetate cross-linked by glutaric aldehyde

| No. | Properties of the resulting product | | | Characteristics of the structure of the obtained product | |
|---|---|---|---|---|---|
| | Content of volatile matter, % by mass | Average particle size, μm | Water-absorption, % by mass | $n=\frac{B+P+D+q}{A}$ | $l=\frac{P+q}{B+D}$ |
| 1 | 2 | 3 | 4 | 5 | 6 |
| 1 | 8 | 150 | 1,500 | 67 | at most 0.001 |
| 2 | 8 | 100 | 650 | 30 | 0.005 |
| 3 | 8 | 300 | 2,000 | 125 | at most 0.176 |
| 4 | 6.5 | 150 | 900 | 30 | 0.030 |
| 5 | 7.8 | 250 | 2,100 | 125 | 0.008 |
| 6 | 8.0 | 200 | 1,900 | 80 | 0.001 |
| 7 | 7.9 | 250 | 1,800 | 71 | 0.104 |
| 8 | 8.1 | 150 | 1,600 | 55 | at most 0.001 |
| 9 | 6.9 | 320 | 2,000 | 125 | at most 0.001 |
| 10 | 8.2 | 200 | 1,800 | 70 | 0.200 |
| 11 | 4.6 | 150 | 700 | 30 | 0.033 |

## CLAIMS :

1. Copolymers of vinyl alcohol with vinylacetate cross-linked by glutaric aldehyde having the structural formula:

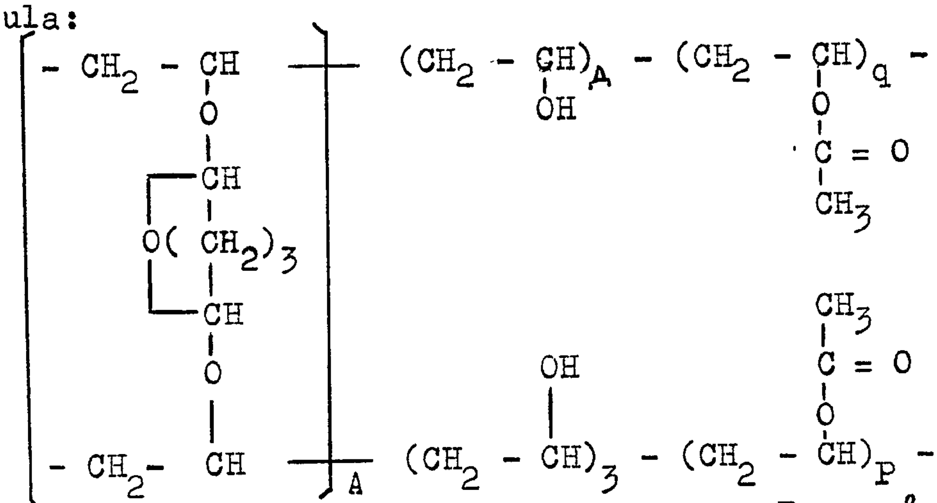

wherein: $\frac{B+P+A+q}{A} = n = 30 \div 125$ and $\frac{P+q}{B+A} = \ell \leq 0.2$

2. A method for preparing copolymers of vinyl alcohol with vinylacetate cross-linked by glutaric aldehyde according to Claim 1 by way of mixing an aqueous solution of polyvinyl alcohol with a content of acetate groups of not more than 20 mol. % with glutaric aldehyde at a temperature of from 15 to 80°C, followed by the addition of mineral acid to a pH of the reaction medium of 1-3, characterized in that the starting components are employed at the ratio of 1,000 molar units of polyvinyl alcohol per 8-40 moles of glutaric aldehyde; cross-linking is conducted under static conditions without stirring; the resulting reaction mass in the form of a hydrogel containing the desired product is disintegrated in a non-hydrating medium, the disintegrated hydrogel is recovered from the obtained suspension, treated in succession with water, a polar solvent and dried.

3. A pharmaceutical preparation exhibiting hemostatic, antiedemic, antiinflammatory and draining effects characterized in that

it consists of an active principle - a copolymer of vinyl alcohol with vinylacetate cross-linked by glutaric aldehyde according to Claim 1.

AMENDED CLAIMS FOR INTERNATIONAL APPLICATION PCT/SU 88/00210

1. Copolymers of vinyl alcohol with vinylacetate cross-linked by glutaric aldehyde having the structural formula:

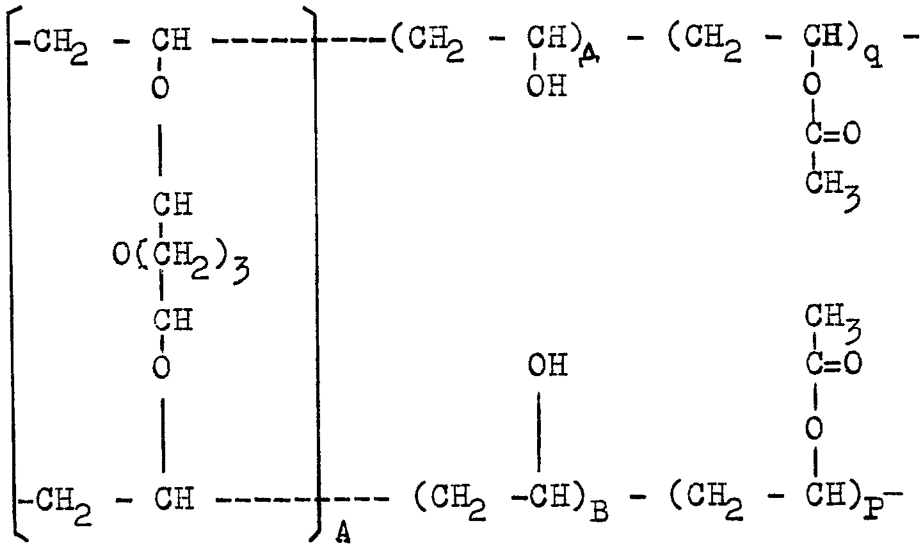

wherein

$$\frac{B + P + A + q}{A} = n = 30\text{-}125 \text{ and } \frac{P + q}{B + A} = \ell \leq 0.2;$$

A = I

B + A = 30-125, A = 15-63, B = 15-63,

P+q = 0 - 25, q = 0-13, P = 0-13.

2. A method for preparing copolymers of vinyl alcohol with vinylacetate cross-linked by glutaric aldehyde according to Claim 1 by way of mixing an aqueous solution of polyvinyl alcohol with a content of acetic groups of at most 20 mol.% with glutaric aldehyde at a temperature of 15-80°C, followed by the addition of a mineral acid to a pH of 1-3 to give a reaction mass, characterized in that the starting components are employed at the ratio of 1,000 molar units of polyvinyl alcohol per 8-40 moles of glutaric aldehyde; cross-linking is conducted under static conditions without stirring; the resulting reaction mass in the form of a hydrogel containing the desired product is disintegrated in a non-hydrating medium, the disintegrated hydrogel is recovered from the obtained suspension, treated in succession with water, a polar solvent and dried.

3. A pharmaceutical preparation exhibiting hemostatic, antiedemic, antiinflammatory and draining effects, characterized in that it consists of an active principle - a copolymer of vinyl acetate cross-linked by glutaric aldehyde according to Claim 1.

# INTERNATIONAL SEARCH REPORT

International Application No PCT/SU 88/00210

**I. CLASSIFICATION OF SUBJECT MATTER** (if several classification symbols apply, indicate all) [6]

According to International Patent Classification (IPC) or to both National Classification and IPC

Int.Cl.$^4$ : C 08 F 216/06, 8/28, A 61 K 31/765, A 61 L 15/03, 15/06

**II. FIELDS SEARCHED**

Minimum Documentation Searched [7]

| Classification System | Classification Symbols |
|---|---|
| Int.Cl.$^4$ | C 08 F 216/06, 8/28, A 61 F 13/18, A 61 K 31/765, A 61 L 15/01, 15/03, 15/06 |

Documentation Searched other than Minimum Documentation to the Extent that such Documents are Included in the Fields Searched [8]

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| A | Plasticheskie massy, Nr 4, April 1988, (Khimia, Moscow), N.V. Meya "Polimery-dlya meditsiny", pages 61-62 | 2 |
| A | Referativny zhurnal "Khimia", Nr 2, 25 January 1981 (25.01.81), (VINITI, Moscow, URSS), Braun D. Intra - und intermolekulare Vernetzung von Polyvinylalkohol mit Dialdehyden c.62, 63, abstract 2 C 350 (colloid and Polym. Sci, 1980, 258, Nr 7, 795-801 (Germ.; summ Engl.) (cited in the application) | 2 |
| A | Referativny zhurnal "Khimia", Nr 20, 1982 (VINITI, Moscow, USSR), Yoshitake Toshihiko et al.; K.K.Kurary, application 56-139580, Japan, filed on 31.03.80, Nr 55-42619, publ. on 31.10.81, IPC : C 09 K 17/00, page 66, abstract 20 C 419 P | 2 |

./.

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 23 June 1989 (23.06.89) | 24 July 1989 (24.07.89) |
| **International Searching Authority** | **Signature of Authorized Officer** |
| ISA/SU | |

International Application No. PCT/SU 88/00210

III. DOCUMENTS CONSIDERED TO BE RELEVANT (CONTINUED FROM THE SECOND SHEET)

| Category * | Citation of Document, with indication, where appropriate, of the relevant passages | Relevant to Claim No |
|---|---|---|
| A | Referativny zhurnal "Khimia", Nr 22, 1982 (VINITI, Moscow, USSR), Yoshitake Toshihiko; K.K.Kuraray, appl. 56-155203, Japan, filed on 30.04.80. Nr 55-58361, publ. on 01.12.81, IPC: C 08 F 8/14, page 76, abstract 22 C 440 P | 2 |
| A | Referatovny zhurnal "Khimia", Nr 17, 1988 (VINITI, Moscow, USSR), Budtov V.P. et al. "Sintez, struktura i svoistva setchatykh polimerov", page 5, abstract 17 C 27 (Tezisy dokl. Vses. konf. Zvenigorod, 1-3 April 1988) | 2 |
| A | Referativny zhurnal "Khimia", Nr 17, 1983 (VINITI, Moscow, USSR), Cholakis Cynthis H. et al. "Chemical characterization of an immobilized heparin: heparin - PVA", c.58, abstract 17 C 343 (Amer. Chem. Sor. Polym. Prepr.", 1983, 24, Nr I, 64-65 (Engl.)) | 2,3 |
| A | US, A, 4262067, (THE UNITED STATES OF AMERICA AS REPRESENTED BY THE ADMINISTRATOR OF THE NATIONAL AERONAUTICS AND SPACE ADMINISTRATION), 14 April 1981 (14.04.81) | 2 |
| A | GB, B, 1567280, (TOYO SEIKAN KAISHA LTD.), 14 May 1980 (14.05.80) | 2 |
| A | III Vsesojuznaya konferentsia "Vodorastvorimye polimery i ikh primenemie", tezisy dokladov, 1987, (Irkutsk), M.I. Gandelsman et al. "Teoreticheskie i experimentalnoe izuchenie nabukhania sshitykh PVS", page 169 | 2 |
| A | DE, AI, 3732642, (EGIS GYOGYSZERGYÁR, MÜANYAGIPARI KUTATO INTÉZET), 07 April 1988 (07.04.88), see the claims | 3 |
| A | EP, AI, 0181970, (SEKISUI KAGAKU KOGYO KABUSHIKI KAISHA), 28 May 1986 (28.05.86) see the claims | 3 |
| A | EP, A2,0200562, (ALZA CORPORATION) 05 November 1986 (05.11.86), see the claims<br>& US, A, 4615699, 07.10.86<br>AU, AI, 5703386, 06.11.86<br>JP, A2, 61-280427, 11.12.86<br>US, A, 4681584, 21.07.87 | 3 |